# EUROPEAN PATENT SPECIFICATION

(11) **EP 2 363 086 B1**
(45) Date of publication and mention of the grant of the patent: **11.06.2014**
(21) Application number: 11466003.8
(22) Date of filing: 02.03.2011
(51) Int. Cl.: A61B 17/70

(54) **Transpedicular reduction screw**
Transpedikularesreposition Schrauben
vis de reduction transpediculaire

(30) Priority: 03.03.2010 CZ 20100154
(43) Date of publication of application: 07.09.2011
(73) Proprietor: Bertagnoli, Rudolf, 1190 Wien (AT)
(72) Inventor: Jirsák, Václav, 12000 Praha 2 (CZ); Srámek, Jiri, 17000 Praha (CZ)
(74) Representative: Skoda, Milan

(56) References cited:
- WO-A1-2009/029928
- WO-A2-2009/106733
- US-A1- 2004 260 283
- US-A1- 2005 131 410
- US-A1- 2007 118 118

## Description

### Technical Field

The invention concerns a fixing device to be used in spine surgery.

### The Existing State of the Art

At the present time, transpedicular fixation of the spine is a standard and dominant method of posterior fixation of the thoracic and lumbar spine, of sacrum and partially also of the cervical spine using smaller implants and CT navigation. The pedicle is a paired cylinder-shaped anatomic structure - the segment between the transverse process and the vertebral body that projects from the supero-dorso-lateral part of the vertebral body in the dorsal or slightly dorso-lateral direction which is, along with the lamina, part of the vertebral arch. The vertebral arch is formed by a pair of pedicles and a pair of laminae, and supports seven processes, four articular, two transverse, and one spinous, the latter also being known as the neural spine. The vertebral arch demarcates the spinal canal with neural structures (spinal cord, nerve roots). The principle of the transpedicular insertion is placing the screw using the posterior approach via the pedicle into the vertebral body. The principle of the transpedicular fixation is connection of heads of individual screws with a fixing rod. The number of connected segments of the spine is not limited. Fixation of the spinal column from the posterior approach using wiring is first described by Hadra. The first author to describe the possibility of placing the screw through the posterior approach was Boucher, but it was only transarticular fixation of one of intervertebral joints (analogous to former transarticular fixation first reported by King or latter translaminar fixation reported by Magerl). Connection of individual segments with transpedicular screws and perforated splints was described by Roy-Camille in the early 60s but it was still about the use of conventional set of orthopaedic instruments. The later Steffee's plate was based just on the Roy-Camille's technique. Another method (that developed partly independently of the development of transpedicular screws) was fixation using two rods that were fixed to the spinal column with either wire loop or sub-laminar hooks. Still another technique that had been developed before the transpedicular fixation was the technique of correction of deformities using the Harrington rod. At the turn of the seventies and eighties of the 20^{th} century, special sets of instruments for transpedicular fixation were developed. At the beginning, attention was turned to development of fixing sets to treat traumatic ruptures which means that various angular-stable implants offering the possibility of reduction in the sagittal plane were developed. These sets of instruments were also developed to facilitate (besides final fixation) the surgical returning of the spine into its physiological shape (reduction). One of the first methods of such reduction described by Daniux used the set of instruments consisting of two unilateral rods - compression one and distraction one. Nevertheless, the use of transpedicular screws, such as Schanz screws, that facilitate good reduction through manipulation with the distal parts of the screws was considered as a real breakthrough. Almost at the same time, external and (especially) internal fixateurs were introduced on which the present-day sets of instruments are based. The standard segmental unit of a modern angular-stable fixateur (offering reduction in the sagittal plane) consists of a fixation rod, a Schanz screw, and of a clamp facilitating arrestment in various positions by means of a congruent tooth system. With the standard diameter of the rod and the Schanz screw being 5 - 7 millimetres, the width of the clamp is approximately 20 mm. The aforementioned is, for example, known from Patent US5047029. If used in the sacrum region, it is often necessary to resect part of the of iliac bone, if used in the thoracic spine region, it is often necessary to resect the transversal or the spinous process (depending on whether the Schanz screw is positioned medially or laterally from the fixation rod) as these anatomic structures may collide with the edge of the clamp. The definite advantage of the aforementioned type of fixation is its high fixation strength. The disadvantage is, besides the above-mentioned size, a difficult way of manipulation and placing the clamps with the rod on the screws if connection of three and more segments is necessary. Unlike the sets of instruments mentioned hereinafter, the clamp with the hole on its antero-posterior axis has to be placed on a greater number of screws which can partly be deflected from the standard plane (which would ideally intersect them) whether by operating surgeon's mistake or due to the specific anatomic situation with a deflection in the frontal plane (scoliosis). In that case, the fixation rod must be perfectly shaped. If the rod is bent, it is very difficult to change the position of the clamp on the rod because the hole for the fixation rod in the clamp has parallel edges and the clamp might get stuck in the place where the rod is bent. Another branch of the development is a group of polyaxial implants suitable for fixation of e.g. degeneration-affected spine without the need for any bigger reduction. The implants are based on the connection of a threaded shaft with the head of the screw on the ball pivot principle. This solution enables the operating surgeon to place the rod on the head of the screws in a very comfortable way even without any need for accurate shaping of the rod. This means that fixation of more segments is very easy. After the fixation rod is inserted, the shaft is fixed in the required position by tightening the arresting screw. Reduction in the sagittal plane which is necessary with affections of the anterior spinal column (e.g. with various traumas) is impossible because no movement of the screw as a whole takes place during manipulation with the head of the screw. The only reduction can be made by pulling the head of the screw along its longitudinal axis (e.g. in treatment for spondylolisthesis). The reduction device is a tube to be pulled over the head of the screw and fixed in the grooves or holes on the side of the head. Any follow-up change in the inclination of the axis in the sagittal plane (if hyper- or hypolordosis occurs in the operated segment) is impossible. Fixation of the head against the shaft of the screw is not as strong as the angular-stable implants which facilitate reduction in the sagittal axis. The reason why the said fixation is not so strong is the fact that it is only achieved through contact between the outer and inner surfaces of two hemispheres. In view of the polyaxial movement, no congruent tooth system can be incorporated into the mechanism (in spite of that it is sometimes incorporated within some mechanisms but when any movement of the ball pivot occurs, the tooth system stops being mutually congruent). The simplest spinal implants are monoaxial screws which facilitate fixation of the rod against the long axis of the screw in the right angle only. Most manufacturers offer a monoaxial screw the head of which has the same design as the head of a polyaxial screw by the same manufacturer. This enables the surgeon to use the same set of instruments. The advantage of monoaxial screws is the high strength of fixation using the rod that is comparable to angular-stable implants which facilitate reduction in the sagittal axis. The disadvantage is their one single position only and the necessity for the follow-up shaping of the rod. From the size of the head point of view, the mono- and polyaxial screws are mutually comparable. The head is mostly cylindrical, 11 to 15 millimetres in diameter and 15 to 20 millimetres in height. Monoaxial screws are roughly 4 mm lower since they do not have the ball-journal mechanism. The cylindrical shape of the head facilitates comfortable placing the monoaxial as well as polyaxial screws in all segments of the thoracic and lumbar spinal column. The fixation rod (in most mono- and polyaxial implants) is inserted into the head of the screw from the above which is technically the simplest way of insertion.

With standard monoaxial and polyaxial screws, the fixation rod in the head of the screw is fixed by a thrust element (which is a screw with a flat ending). The contact surface between the standard fixation rod with a circular cross-section and the thrust element is in the shape of just a line segment. A certain improvement is offered in the design described in patent application US 2004/260283. It uses a system of three thrust elements which are in contact with larger surfaces. The fixation rod, however, is mounted in the only possible position against the head of the screw. Such a design therefore does not make possible any swinging movement against the head of the screw which means that it does not allow any reduction in the sagittal plane.

It follows from the state of the art published that, with implants whose head sizes correspond to monoaxial and polyaxial screws, there exist designs of transpedicular reduction screws described in patent applications WO 2009/106733, WO 2009/029928 and US 2007/0118118. Here, the screws have two thrust elements in the upper part of the arresting mechanism between which biaxial movement takes place (positioning the fixation rod and the lower thrust element against the head of the screw and tightening the upper thrust element by rotation on the longitudinal axis of the head of the screw). This means that the contact surface is spherical, and it is impossible to incorporate into it any congruent tooth system which results in the strength of the fixation being considerably lower in comparison with systems using a clamp and the Schanz screw.

### The Nature of the Invention

The aforementioned weaknesses are, to a large extent, eliminated and the goals of the invention accomplished by a transpedicular reduction screw, especially a transpedicular angular-stable reduction screw with an arresting mechanism of the fixation rod arranged inside the head of the screw according to the invention whose principle lies in the fact that the arresting mechanism of the rod consists of its lower part and its upper part which includes three thrust elements arranged in such a manner that the lower thrust element (mounted on the fixation rod) is pressed down by the middle thrust element which is pressed down by the upper thrust element fixed in the head of the screw, and one side of the the fixation rod is placed in the lower part and the other side in the upper part so that the lower part, the fixation rod and the lower thrust element can swing about the "y" axis in respect to the head of the screw, the middle thrust element and the upper thrust element.

It is advantageous, if the upper thrust element is fixed in the threaded walls of the head of the screw. This facilitates quick assembly of the whole set of the transpedicular reduction screw. Another simplification of the assembly process is brought by the fixation rod being placed rotatably inside the arresting mechanism.

In order to prevent undesirable rotation against the head of the screw around the "z" axis, it is advisable that the lower thrust element in the upper part of the arresting mechanism is rectangular in its ground plan with its longer side exceeding the diameter of the upper thrust element.

To assure the stable arresting of individual parts of the transpedicular reduction screw in the functional state, it is advisable that the contact surfaces between the head of the screw and the lower part and/or between the lower part and the fixation rod and/or between the lower thrust element and the fixation rod and/or between the lower thrust element and the middle thrust element are provided with teeth.

Ideally, to make the assembly easier and quicker, the upper thrust element should be provided with a hole for the assembly instrument in its upper surface. It is advantageous if the hole is in the shape of the Phillips drive or if it can accommodate a hexagonal or torx inner wrench.

To prevent mutual movements (except for rotation and shift in the "z" axis), it is advisable that the upper surface of the middle thrust element is provided with a circular groove to accommodate the circular prominence on the lower surface of the upper thrust element or (optionally) vice versa.

The assembly of the set and manipulation with it is even easier if the lower part of the arresting mechanism is rotatably connected with the head of the screw using a connecting part placed in the "z" axis on the understanding that the connecting part passes through an opening made in the lower part of the arresting mechanism.

It is advantageous if the lower thrust element, the middle thrust element and the upper thrust element are mutually connected using the upper connecting part freely arranged in the openings that have been made in the axis of the lower thrust element, middle thrust element and of the upper thrust element in such a manner that the lower thrust element can rotate against the middle thrust element around the "y" axis and the upper thrust element can rotate against the middle thrust element around the "z" axis. It is advisable that the ends of the upper connecting part are provided with stops to prevent the lower, middle and upper thrust elements from mutual disconnection.

The advantageous variant has the upper connecting part firmly connected to the middle thrust element or to the upper thrust element. This solution makes the assembly easier and quicker.

The rod can rotate around the "y" axis owing to the cylindrical or spherical contact surfaces between the lower part of the arresting mechanism and the head of the screw and/or between the lower thrust element and the middle thrust element.

The transpedicular reduction screw according to this invention is an angular-stable reduction screw intended for fixation of the thoracic and lumbar spine. The head and the rod of the screw form one rigid block. The arresting mechanism is found in the opening inside the head of the screw. The set of the elements that form the lower part of the arresting mechanism can rotate against the head of the screw around the "y" axis all the way to the contact point between the fixation rod and the head of the screw. The fixation rod can rotate freely around the "x" axis. The fixation rod can easily be inserted into the hole in the head of the screw from the above thus facilitating easier multi-segment reduction and stabilization.

The transpedicular angular-stable reduction screw with the arresting mechanism inside the head of the screw that is the subject matter of this invention facilitates reduction of the spin in the sagittal plane which is impossible with a standard polyaxial screw. In comparison with the designs described in patent applications WO 2009/106733. WO 2009/029928 and US 2007/0118118, it offers considerably higher strength of stabilization because the contact surfaces of the thrust elements can be profiled and/or provided with a congruent tooth system..

In comparison with the systems incorporating the Schanz screw, our screw is axially symmetrical and does not protrude to the sides. When implanted in the sacral vertebra (S1), it does not collide with the wing of ilium and when implanted in the thoracic spine, it does not collide with transversal or spinal processes. In addition, it facilitates easy manipulation and simple insertion of the fixation rod in the head of the screw as it eliminates two operations: insertion of the rod in the clamp followed by putting the clamp on the Schanz screw. Thus, it further facilitates connection of more than two screws. In this way, the transpedicular angular-stable reduction screw with the arresting mechanism inside the head of the screw that is the subject matter of this invention combines the advantages of fixation offered by the systems with the Schanz screws and the advantages of shapes and sizes of the head offered by mono- and polyaxial screws.

### Outline of Individual Figures on the Drawing

The nature of the invention is better clarified on the drawing where Fig. 1 shows an expanded axonometric view of the general arrangement of the transpedicular reduction screw with smooth contact surfaces, Fig. 2 shows an expanded axonometric view of the general arrangement of the transpedicular reduction screw with some contact surfaces provided with teeth, and Fig. 3 shows an expanded axonometric view of the general arrangement of the transpedicular reduction screw with some contact surfaces provided with teeth and with the use of the aforedescribed connecting parts.

### Examples of Individual Designs of the Invention

### Example 1

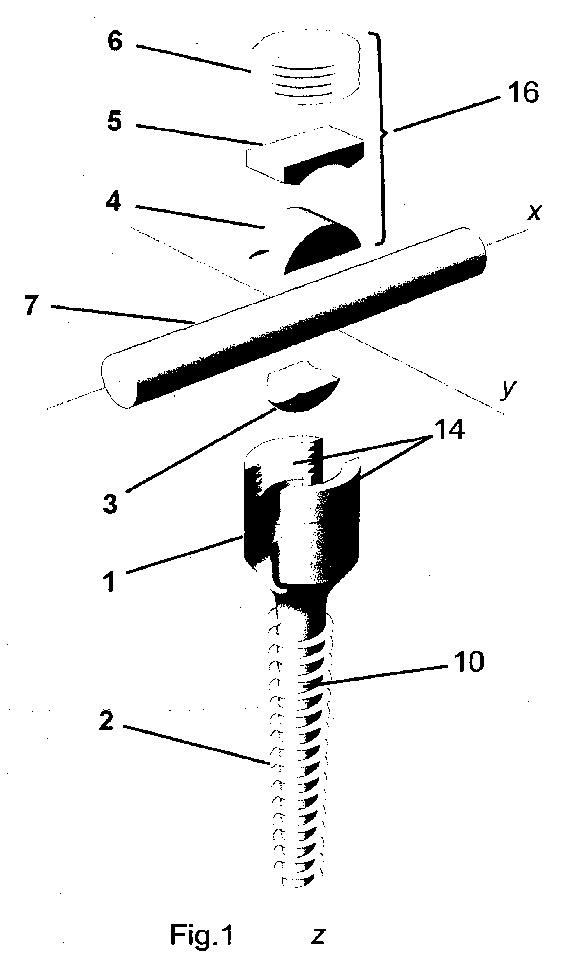

The transpedicular angular-stable reduction screw (Fig. 1) includes the screw 10 consisting of the head 1 and the shaft 2. It further includes the arresting mechanism of the fixation rod 7 arranged inside the head 1.

The arresting mechanism consists of the lower part 3 and the upper part 16. The fixation rod 7 is, from one side, placed in the lower part 3 placed inside the head 1 of the screw 10 and, from the other side, in the lower thrust element 4 of the upper part 16 on which the middle thrust element 5 of the upper part 16 is seated. This thrust element 5 is pressed down by the upper thrust element 6 fixed in the head 1 of the screw 10. The fixation rod 7 and the lower thrust element 4 can swing about the "y" axis in respect to the head of the screw 1, the middle thrust element 5 and the upper thrust element 6.

The lower part 3 is mounted swingably inside the head 1 of the screw 10. The lower thrust element is also mounted swingably inside the middle thrust element 5.

The upper thrust element 6 is fixed in the threaded walls 14 of the head 1 of the screw 10.

The fixation rod 7 is mounted rotatably in the arresting mechanism.

The ground plan of the lower thrust element 4 of the upper part 16 of the arresting mechanism is rectangular the longer side of which exceeds the diameter of the upper thrust element 6.

The contact surface between the lower part 3 of the arresting mechanism and the head 1 of the screw 10 is cylindrical or spherical. The contact surface between the lower thrust element 4 and the middle thrust element 5 is cylindrical. All the contact surfaces are smooth.

During operation, the screw 10 is screwed through the pedicle into the body of the vertebra it being understood that two screws 10 are to be screwed into one vertebra. Then, the vertebrae intended for fusion are to be connected using the fixation rod 7 and arrested in the desired position.

### Example 2

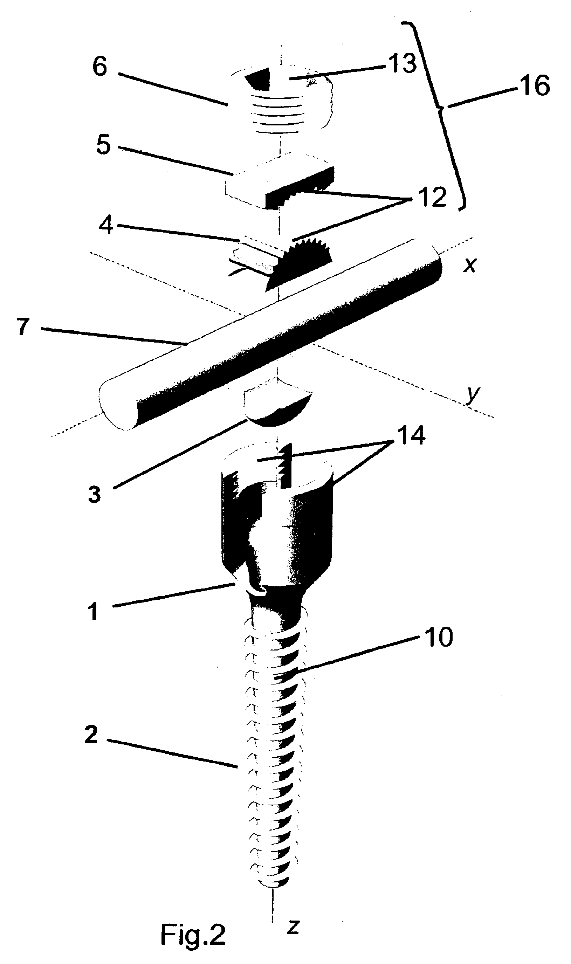

The transpedicular angular-stable reduction screw (Fig. 2) includes the screw 10 consisting of the head 1 and the shaft 2. It further includes the arresting mechanism of the fixation rod 7 arranged inside the head 1.

The arresting mechanism consists of the lower part 3 and the upper part 16. The fixation rod 7 is, from one side, placed in the lower part 3 placed inside the head 1 of the screw 10 and, from the other side, in the lower thrust element 4 of the upper part 16 on which the middle thrust element 5 of the upper part 16 is seated This thrust element 5 is pressed down by the upper thrust element 6 fixed in the head 1 of the screw 10. The fixation rod 7 and the lower thrust element 4 can swing about the "y" axis in respect to the head of the screw 1, the middle thrust element 5 and the upper thrust element 6.

The lower part 3 is mounted swingably inside the head 1 of the screw 10. The lower thrust element is also mounted swingably inside the middle thrust element 5.

The upper thrust element 6 is fixed in the threaded walls 14 of the head 1 of the screw 10.

The fixation rod 7 is mounted rotatably in the arresting mechanism.

The ground plan of the lower thrust element 4 of the upper 16 part of the arresting mechanism is rectangular the longer side of which exceeds the diameter of the upper thrust element 6.

The contact surfaces between the lower thrust element 4 and the middle thrust element 5 are provided with teeth 12. As the case may be, the contact surfaces between the head 1 of the screw 10 and the lower part 3 and between the lower part 3 and the fixation rod 7 and between the lower thrust element 4 and the fixation rod 7 may also be provided with teeth.

The upper thrust element 6 is, in its upper surface, provided with a hexagonal drive 13 for the respective assembly tool (inner wrench).

The contact surface between the lower thrust element 4 and the middle thrust element 5 is cylindrical. The contact surface between the lower part 3 of the arresting mechanism and the head 1 of the screw 10 is cylindrical or spherical and made as a smooth one.

During operation, the screw 10 is screwed through the pedicle into the body of the vertebra it being understood that two screws 10 are to be screwed into one vertebra. Then, the vertebrae intended for fusion are to be connected using the fixation rod 7 and arrested in the desired position.

### Example 3

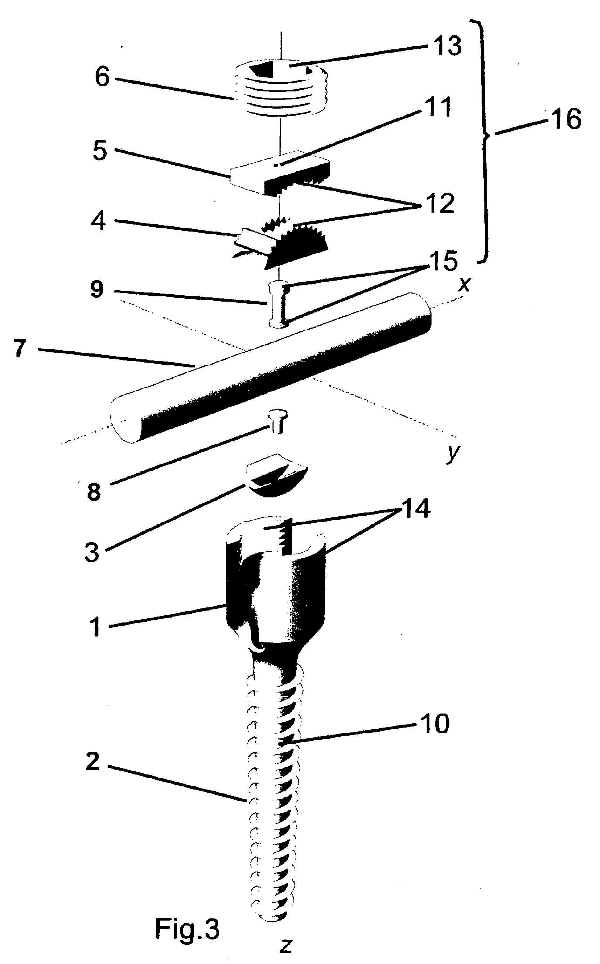

The transpedicular angular-stable reduction screw (Fig. 3) includes the screw 10 consisting of the head 1 and the shaft 2. It further includes the arresting mechanism of the fixation rod 7 arranged inside the head 1.

The arresting mechanism consists of the lower part 3 and the upper part 16. The fixation rod 7 is, from one side, placed in the lower part 3 placed inside the head 1 of the screw 10 and, from the other side, in the lower thrust element 4 of the upper part 16 on which the middle thrust element 5 of the upper part 16 is seated. This thrust element 5 is pressed down by the upper thrust element 6 fixed in the head 1 of the screw 10. The fixation rod 7 and the lower thrust element 4 can swing about the "y" axis in respect to the head of the screw 1, the middle thrust element 5 and the upper thrust element 6.

The lower part 3 is mounted swingably inside the head 1 of the screw 10. The lower thrust element is also mounted swingably inside the middle thrust element 5.

The upper thrust element 6 is fixed in the threaded walls 14 of the head 1 of the screw 10.

The fixation rod 7 is mounted rotatably in the arresting mechanism.

The ground plan of the lower thrust element 4 of the upper part 16 of the arresting mechanism is rectangular the longer side of which exceeds the diameter of the upper thrust element 6.

The contact surfaces between the lower thrust element 4 and the middle thrust element 5 are provided with teeth 12.

The upper thrust element 6 is, in its upper surface, provided with a hexagonal drive 13 for the respective assembly tool (inner wrench).

In the upper surface of the middle thrust element 5 is a circular groove (not shown) to fit the circular prominence on the lower surface of the upper thrust element 6 and (optionally) vice versa.

The lower part 3 of the arresting mechanism is rotatably connected with the head 1 of the screw 10 using the lower connecting part 8 placed in the "z" axis and firmly fixed to the head 1 of the screw 10. The connecting part 8 passes through an opening made in the lower part 3 of the arresting mechanism.

The lower thrust element 4, the middle thrust element 5 and the upper thrust element 6 are mutually connected using the upper connecting part 9 freely arranged in the openings 11 that have been made in the axis of the lower thrust element 4, middle thrust element 5 and of the upper thrust element 6 in such a manner that the lower thrust element 4 can rotate against the middle thrust element 5 around the "y" axis and the upper thrust element 6 can rotate against the middle thrust element 5 around the "z" axis. The ends of the upper connecting part 9 are provided with stops 15 to prevent the lower thrust element 4, the middle thrust element 5 and the upper thrust element 6 from mutual disconnection. The upper connecting part can optionally be firmly fixed to the middle thrust element 5 or to the upper thrust element 6.

The contact surface between the lower part 3 of the arresting mechanism and the head 1 of the screw 10 is cylindrical or spherical. The contact surface between the lower thrust element 4 and the middle thrust element 5 is cylindrical and is provided with teeth 12.

During operation, the screw 10 is screwed through the pedicle into the body of the vertebra it being understood that two screws 10 are to be screwed into one vertebra. Then, the vertebrae intended for fusion are to be connected using the fixation rod 7 and arrested in the desired position.

### Industrial Utility

The transpedicular reduction screw, especially the transpedicular angular-stable reduction screw with the arresting mechanism inside the head of the screw is intended to be used in the field of spine surgery.

### List of Reference Items

- 1: Head
- 2: Shaft
- 3: Lower Part
- 4: Lower Thrust Element
- 5: Middle Thrust Element
- 6: Upper Thrust Element
- 7: Fixation Rod
- 8: Connecting part
- 9: Upper connecting part
- 10: Screw
- 11: Hole, Opening
- 12: Teeth, Tooth System
- 13: Hole (Drive) for Assembly Tool
- 14: Wall
- 15: Stop
- 16: Upper part

## Claims

1. A transpedicular reduction screw, especially a transpedicular angularstable reduction screw with an arresting mechanism of a fixation rod (7) placed inside the head (1) of the screw (10), the arresting mechanism of the fixation rod (7) including a lower part (3), **characterized in that** an upper part (16) which has three thrust elements (4, 5, 6) arranged in such a manner that the lower thrust element (4) mounted on the fixation rod (7) is pressed down by the middle thrust element (5) which is further pressed down by the upper thrust element (6) fixed in the head (1) of the screw (10) with one side of the fixation rod (7) being mounted in the lower part (3) and with the other side of the fixation rod being mounted in the upper part (16) in such a manner that the lower part (3), the fixation rod (7) and the lower thrust element (4) can swing about the "y" axis in respect to the head of the screw (1), the middle thrust element (5) and the upper thrust element (6).

2. The transpedicular reduction screw according to claim 1 **characterized in that** the contact surfaces of the lower part (3) of the arresting mechanism and of the head (1) of the screw (10) and/or of the lower thrust element (4) and of the middle thrust element (5) are cylindrical or spherical.

3. The transpedicular reduction screw according to some of the previous claims **characterized in that** the upper thrust element (6) is fixed in the threaded walls (14) of the head (1) of the screw (10).

4. The transpedicular reduction screw according to some of the previous claims **characterized in that** the fixation rod (7) is placed rotatably inside the arresting mechanism.

5. The transpedicular reduction screw according to some of the previous claims **characterized in that** the ground plan of the lower thrust element (4) of the upper part (16) of the arresting mechanism is rectangular the longer side of which exceeds the diameter of the upper thrust element (6).

6. The transpedicular reduction screw according to some of the previous claims **characterized in that** the contact surfaces between the head (1) of the screw (10) and the lower part (3) and/or between the lower part (3) and the fixation rod (7) and/or between the lower thrust element (4) and the fixation rod (7) and/or between the lower thrust element (4) and the middle thrust element (5) are provided with teeth (12).

7. The transpedicular reduction screw according to some of the previous claims **characterized in that** the upper thrust element (6) is, in its upper surface, provided with a hole for the respective assembly tool.

8. The transpedicular reduction screw according to some of the previous claims **characterized in that** the upper surface of the middle thrust
element (5) is provided with a circular groove to fit the circular prominence on the lower surface of the upper thrust element (6) or the lower surface of the upper thrust element (6) is provided with a circular groove to fit the circular prominence on the upper surface of the middle thrust element (5).

9. The transpedicutar reduction screw according to some of the previous claims **characterized in that** the lower part (3) of the arresting mechanism is rotatably connected with the head (1) of the screw (10) using the lower connecting part (8) placed in the "z" axis and firmly fixed to the head (1) of the screw (10) it being understood that the connecting part (8) passes through an opening made in the lower part (3) of the arresting mechanism.

10. The transpedicular reduction screw according to some of the previous claims **characterized in that** the lower thrust element (4), the middle thrust element (5) and the upper thrust element (6) are mutually rotatably connected using the upper connecting part (9) freely arranged in the openings that have been made in the axis of the lower thrust element (4), of the middle thrust element (5) and of the upper thrust element (6) in such a manner that the lower thrust element (4) can rotate against the middle thrust element (5) around the "y" axis and the upper thrust element (6) can rotate against the middle thrust element (5) around the "z" axis with the ends of the upper connecting part (9) being provided with stops (15) to prevent the lower thrust element (4), the middle thrust element (5) and the upper thrust element (6)
from mutual disconnection.

11. The transpedicular reduction screw according to claim 10 **characterized in that** the upper connecting part (9) is firmly fixed to the middle thrust element (5) or to the upper thrust element (6).

## Patentansprüche

1. Die transpedikuläre Repositionsschraube, vor allem eine transpedikuläre winkelstabile Repositionsschraube mit einem Arretiermechanismus des Fixationsstabes (7), angebracht im Kopf(1) der Schraube (10), wo der Arretiermechanismus des Fixationsstabes (7) den unteren Teil (3) einschließt, ist **dadurch gekennzeichnet, dass** der obere Teil (16) drei Andruckelemente (4,5,6) enthält, die so angeordnet sind, das das untere auf dem Fixationsstab (7) befestigte Andruckelement (4) durch das mittlere Andruckelement (5) heruntergedrückt wird und zusätzlich durch das obere Andruckelement (6) heruntergedrückt wird, welches im Kopf (1) der Schraube (10) fixiert ist, wobei der Fixationsstab (7) mit einer Seite in dem unteren Teil (3) und aus der anderen Seite in dem oberen Teil (16) so gelagert ist, dass der untere Teil (3), den Fixationsstab (7) sowie das untere Andruckelement (4) gegenüber dem Schraubenkopf (1), dem mittleren Andruckelement (5) und dem oberen Andruckelement (6) um die Achse "y" schwenkbar gelagert sind.

2. Die transpedikuläre Repositionsschraube, nach dem Patentanspruch 1 ist **dadurch gekennzeichnet, dass** die Kontaktflächen des unteren Teils (3) des Arretiermechanismus und des Kopfes (1) der Schraube (10), und/oder jene des unteren Andruckelementes (4) sowie jene des mittleren Andruckelementes (5) zylinderförmig oder kugelförmig sind.

3. Die transpedikuläre Repositionsschraube ist , gemäß nach einigen der oben erwähnten Patentansprüche, **dadurch gekennzeichnet, dass** das obere Andruckelement (6) in den Wandflächen (14) des Kopfes (1) der Schraube (10) gewindeartig befestigt ist.

4. Die transpedikuläre Repositionsschraube ist, gemäß nach einigen der oben erwähnten Patentansprüche, **dadurch gekennzeichnet, dass** die Fixationsstange (7) in dem Arretiermechanismus drehbar gelagert ist.

5. Die transpedikuläre Repositionsschraube ist, gemäß nach einigen der oben erwähnten Patentansprüche, **dadurch gekennzeichnet, dass** das untere Andruckelement (4) des oberen Teiles (16) vom Arretiermechanismus im Grundriss die Form eines Rechteckes hat, dessen längere Seite größer als der Durchmesser des oberen Andruckelementes (6) ist.

6. Die transpedikuläre Repositionsschraube ist, gemäß nach einigen der oben erwähnten Patentansprüche, **dadurch gekennzeichnet, dass** die Kontaktflächen des Kopfes (1) der Schraube (10) und des unteren Teiles (3) und/oder des unteren Teiles (3) und des Fixationsstabes (7) und/oder jene des unteren Andruckelementes (4) und des Fixationsstabes (7) und/oder jene des unteren Andruckelementes (4) und des mittleren Andruckelementes (5) mit einer Verzahnung (12) versehen sind.

7. Die transpedikuläre Repositionsschraube ist , gemäß nach einigen der oben erwähnten Patentansprüche, **dadurch gekennzeichnet, dass** das obere Andruckelement (6) auf seiner oberen Fläche mit einer Öffnung (13) für das entsprechende Montagewerkzeug versehen ist.

8. Die transpedikuläre Repositionsschraube ist , gemäß nach einigen der oben erwähnten Patentansprüche, **dadurch gekennzeichnet, dass** auf der oberen Fläche des mittleren Andruckelementes (5) eine kreisförmige Nut angeordnet ist, in welche eine kreisförmige Vorwölbung der unteren Fläche des oberen Andruckelementes (6) eingreift oder die kreisförmige Nut ist auf der unteren Fläche des oberen Andruckelementes (6) und greift in die kreisförmige Vorwölbung der oberen Fläche des mittleren Andruckelementes (5) ein.

9. Die transpedikuläre Repositionsschraube ist , gemäß nach einigen der oben erwähnten Patentansprüche **dadurch gekennzeichnet, dass** der untere Teil (3) des Arretiermechanismus mit dem Kopf (1) der Schraube (10) über den unteren Verbindungsteil (8) drehbar verbunden ist; dieser befindet sich an der Achse "z" und ist mit dem Kopf (1) der Schraube (10) fest verbunden, wobei der Verbindungsteil (8) in eine im unteren Teil (3) des Arretiermechanismus befindliche Öffnung durchgeführt wird.

10. Die transpedikuläre Repositionsschraube ist , gemäß nach einigen der oben erwähnten Patentansprüche **dadurch gekennzeichnet, dass** das untere Andruckelement (4), das mittlere Andruckelement (5) und das obere Andruckelement (6) mit dem oberen Verbindungsteil (9) gegenseitig drehbar verbunden sind, dadurch dass das obere Verbindungsteil (9) frei beweglich in den Öffnungen befindlich in der Achse des unteren Andruckelements (4), des mittleren Andruckelements (5) und des oberen Andruckelements (6) rotieren kann in der Weise das das untere Andruckelement (4) gegen das mittlere Andruckelement (5) um die Achse "y" und das oberen Andruckelement (6) rotieren kann und das obere Andruckelement (6) gegen das mittlere Andruckelement (5) um die Achse "z" mit den Enden des oberen Verbindungsteils (9) rotiert. Diese ist mit Stopps (15) ausgestattet um zu verhindern das das untere Andruckelement (4), das mittlere Andruckelement (5) und das obere Andruckelements (6) die gegenseitige Verbindung trennen können.

11. Die transpedikuläre Repositionsschraube ist nach dem Patentanspruch 10 **dadurch gekennzeichnet, dass** der obere Verbindungsteil (9) zu dem mittleren Andruckelement (5) oder dem oberen Andruckelement (6) fest fixiert ist.

## Revendications

1. Vis transpédiculaire de reposition, surtout la vis transpédiculaire de reposition à stabilité angulaire avec un mécanisme d'arrêtage de la tige de fixation (7), se trouvant à l'intérieur de la tête (1) de vis (10), où le mécanisme d'arrêtage de la tige de fixation (7) comprend la partie inférieure (3), **caractérisé en ce que**, la partie supérieure (16) comprend trois éléments de pression (4,5,6) arrangés comme suit, l'élément de pression inférieur (4), logé sur la tige de fixation (7), est serré par l'élément de pression moyen (5), ce dernier étant serré par l'élément de pression supérieur (6) fixé à la fois dans la tête (1) de vis (10), tandis que la tige de fixation (7) se trouve logée, d'un côté, dans la partie inférieure (3) et de l'autre côté dans la partie supérieure (16) d'une telle façon que la partie inférieure (3), la tige de fixation (7) et l'élément de pression inférieur (4) soient inclinables autour de l'axe "y" par rapport à la tête de vis (1), à l'élément de pression moyen (5) et à l'élément de pression supérieur (6).

2. Vis transpédiculaire de reposition, conforme au droit 1, **caractérisé en ce que**, les surfaces de contact de la partie inférieure (3) du mécanisme d'arrêtage et de la tête (1) de vis (10), et/ou celles de l'élément de pression inférieur (4) et de l'élément de pression moyen (5) sont cylindriques ou sphériques.

3. Vis transpédiculaire de reposition, conforme à un des droits précités, **caractérisé en ce que**, l'élément de pression supérieur (6) est fixé par un filetage dans les parois (14) de la tête (1) de vis (10).

4. Vis transpédiculaire de reposition, conforme à un des droits précités, **caractérisé en ce que**, la tige de fixation (7) est logée dans le mécanisme d'arrêtage de la manière rotative.

5. Vis transpédiculaire de reposition, conforme à un des droits précités, **caractérisé en ce que**, dans sa projection horizontale, l'élément de pression inférieur (4) de la partie supérieure (16) du mécanisme d'arrêtage est de la forme d'un rectangle dont le côté plus long dépasse le diamètre de l'élément de pression supérieur (6).

6. Vis transpédiculaire de reposition, conforme à un des droits précités, **caractérisé en ce que**, les surfaces de contact de la tête (1) de vis (10) et de la partie inférieure (3) et/ou celles de la partie inférieure (3) et de la tige de fixation (7) et/ou celles de l'élément de pression inférieur (4) et de la tige de fixation (7) et/ou celles de l'élément de pression inférieur (4) et de l'élément de pression moyen (5) possèdent une denture (12).

7. Vis transpédiculaire de reposition, conforme à un des droits précités, **caractérisé en ce que**, dans la surface supérieure de l'élément de pression supérieur (6), il y a une ouverture (13) pour l'outil de montage.

8. Vis transpédiculaire de reposition, conforme à un des droits précités, **caractérisé en ce que**, dans la surface supérieure de l'élément de pression moyen (5), il y a une rainure circulaire, dans laquelle engrène la saillie ronde de la surface inférieure de l'élément de pression supérieur (6), ou bien la rainure circulaire se trouve dans la surface de l'élément de pression supérieur (6) pour accueillir la saillie ronde de la surface supérieure de l'élément de pression moyen (5).

9. Vis transpédiculaire de reposition, conforme à un des droits précités, **caractérisé en ce que**, la partie inférieure (3) du mécanisme d'arrêtage est liée de la manière rotative à la tête (1) de vis (10) de la pièce d'assemblage inférieure (8), située sur l'axe "z" et liée de la manière rigide à la tête (1) de vis (10), la pièce d'assemblage (8) étant insérée dans une ouverture faite dans la partie inférieure (3) du mécanisme d'arrêtage.

10. Vis transpédiculaire de reposition, conforme à un des droits précités, **caractérisé**
**en ce que**, l'élément de pression inférieur (4), l'élément de pression moyen (5) et l'élément de pression supérieur (6) sont liés entre eux par la pièce d'assemblage supérieure (9) mise librement dans les ouvertures situées dans l'axe des éléments de pression inférieur (4), moyen (5) et supérieur (6) d'une telle façon que l'élément de pression inférieur (4) puisse se tourner autour de l'axe "y" par rapport à l'élément de pression moyen (5) et que l'élément de pression supérieur (6) puisse se tourner autour de l'axe "z" par rapport à l'élément de pression moyen (5), tandis que les taquets d'arrêt (15), montés aux extrémités de la pièce d'assemblage supérieure (9), empêchent le désassemblage des éléments de pression inférieur (4), moyen (5) et supérieur (6).

11. Vis transpédiculaire de reposition, conforme au droit 10, **caractérisé en ce que**, la pièce d'assemblage supérieure (9) est fixée de la manière rigide à l'élément de pression moyen (5) ou à l'élément de pression supérieur (6).
